# EUROPEAN PATENT APPLICATION

(11) **EP 4 582 809 A1**
(43) Date of publication of application: **09.07.2025**
(21) Application number: 24020004.8
(22) Date of filing: 05.01.2024
(51) Int. Cl.: G01N 33/545, G01N 33/74

(54) **BIOSENSOR AND PRODUCTION THEREOF**

(71) Applicant: Nutrix AG, 4056 Basel (CH)
(72) Inventor: NEUHAUS, Sonja, 5102 Rupperswil (CH); SANVITO, Kay Simon, 5200 Brugg (CH); GOEL, Alok, 8006 Zürich (CH); PARMAR, Jemish Mahendrabhai, 08015 Barcelona (ES)
(74) Representative: Pietruk, Claus Peter

(57) **Abstract**

A method of producing a biosensor or a part thereof is disclosed. The biosensor or part thereof comprises a porous polymer; biomolecules suitable for reaction with an analyte to be sensed by said biosensor, said biomolecules being immobilized by the polymer matrix; a carrier for the polymer matrix, the carrier allowing read-out of reactions of the biomolecules with an analyte. The method disclosed comprises the steps of providing a polymer solution; adding the biomolecules to the solution in defined quantities; dispensing a specific volume of the solution with the added biomolecules onto the carrier; subjecting the solution comprising the added biomolecules and dispensed on the carrier to an ebeam and preferably to drying to remove any solvent and to thus immobilize the biomolecules.

## Description

The present invention generally relates to the detection of substances in body fluids.

In a number of cases, it is desirable to monitor physiological conditions of a living being by monitoring the concentration, presence or absence of certain analytes. This may help to administer pharmaceuticals at the right time and/or in the right concentration, to call an emergency, inform the user of a medical condition and so forth. To allow such monitoring, reliable biosensors are needed and the production of such biosensors should be possible in a cost-effective manner.

Regarding reliability of the monitoring by such sensors, it has been suggested to use substances found e.g. in body fluids as analytes and to effect and detect a reaction between such substances and biomolecules able to react therewith, such as antibodies for these analytes. The measurement can be either invasive, that is a device is placed inside the human body such as the mouth, or non-invasive, that is the body fluid is obtained from the body and subjected to the measurement outside the body. In the later case, the body fluid could e.g. be blood, which however may be painful to retrieve, or urine, saliva, sperm, sweat, tears, sputum asf., which can be retrieved more easily. In such a non-invasive measurement, the body fluid can be brought into contact with the antibodies or other reactive substances and the reaction can then be detected in any known method. Sensitive methods of measurements comprise e.g. optical or electrochemical measurements. In an electrochemical measurement, the biomolecules able to react with the analytes in the body fluid can be immobilized on electrodes and the body fluid with the analytes is brought into contact with these biomolecules.

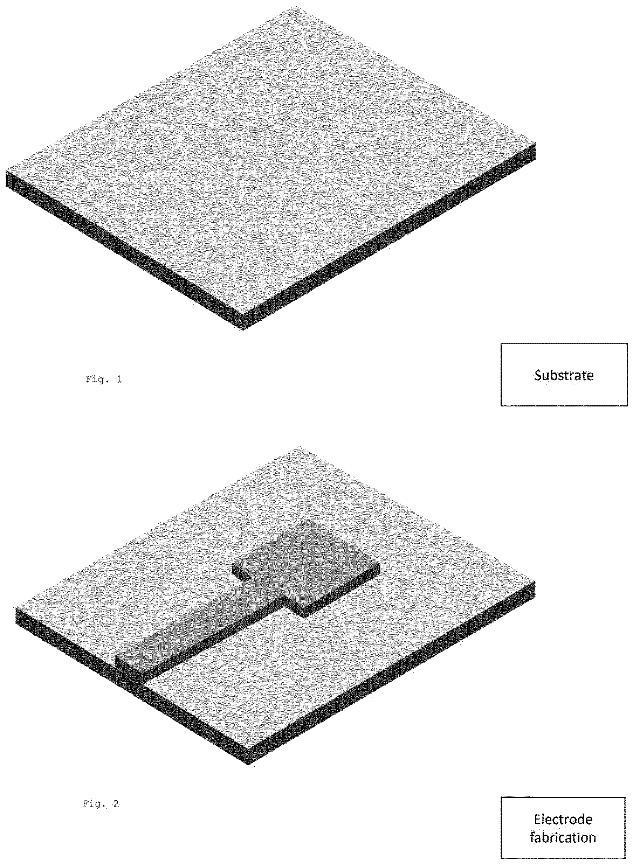

As an example, electrodes could be functionalised for cortisol biosensing in biofluids such as saliva, sweat or blood. This has been suggested for example in "Electrochemical sensors for cortisol detections: Almost there" by Miguel Zea et al. in Trends in Analytical Chemistry 132 (2020) 116058 where in Fig. 2, an example of an antibody functionalized electrode and its electrochemical response toward cortisol by electrochemical impedance spectroscopy is shown, suggesting a stepwise fabrication of an immunosensor in that a bare electrode is provided and then prepared for the reception of antibodies said to generally have carboxylic groups and subsequent immobilization of the antibody, so that of cortisol molecules can be detected using impedimetric measurement for cortisol detection.

While this sounds straightforward, a number of problems arise when such a method of producing a biosensor is to be implemented in a commercial product which is to be used by a person not experienced with laboratory techniques. Among the problems that need to be solved is the necessity to immobilize the antibodies or other biomolecules, to allow for a sufficiently sensitive measurement, to allow for a sufficiently selective measurement not or hardly effected by cross-sensitivies in particular cross-sensitivies to molecules other than the analyte to be found in the respective body fluid, the variation of sensitivity over different batches, the shelf-life of a product, where only a shelf-life of e.g. at least several days, preferably weeks or months after manufacturing considered acceptable. Problems become even more pronounced where it must be taken into account that a person measuring an analyte in saliva might take the device or a part thereof, such as a measuring strip, directly into the mouth, necessitating that the person will not be harmed by any of the chemicals used; or where the same coated electrodes are to be used repeatedly, as it might then be necessary to prevent fouling of the surface caused for example by growth of bacteria due to protein residues from a previous measurement.

A number of attempts have been made to tackle these problems, but these attempts have not been entirely successful thus far.

These problems hold even for the above mentioned cortisol despite being a favorable molecule in that it can diffuse easily while unwanted molecules present in e.g. saliva are typically larger and thus can be more easily prevented from reaching the biomolecules.

One approach has been suggested in WO 2013/101 855 A1 relating to porous membranes permanently grafted with a polymeric coating to facilitate the immobilization of a biomolecule on the porous membrane. Methods of preparing and using the modified porous membranes with these polymeric coatings are described in the prior art document. In one example therein, the modified porous membrane is suggested to comprise a polymer coating of at least one epoxy group-containing compound. The epoxy group-containing compound is suggested to be grafted to the porous membrane by generating free radicals on the porous membrane using e-beam irradiation and a polymer of variable length is to be used that contains an e-beam reactive moiety, designated as "poly-(A) x polymer," as well as a "linkage" that forms a bond between the poly-(A)x polymer and a functional group that is able to react with a chemical group present on a biomolecule, so as to result in the formation of a polymer coating of, for example, an epoxy group-containing compound, such as GMA, permanently bound to the porous membrane. In the prior art document, "e-beam reactive moiety," is considered to be any chemical functional group that is believed to self-polymerize when subjected to e- beam irradiation. Exemplary e-beam reactive moieties include but are not limited to those compounds that comprise a methacrylate, an acrylate, an acrylamide, a vinyl ketone, a styrenic, a vinyl ether, a vinyl-containing moiety, an allyl-containing moiety, a benzyl-based compound, and a tertiary-carbon (CHR3)-based compound, or mixtures of the e-beam reactive moieties set forth above. Note that such e-beam reactive moiety might be used as "polymers" in the context of the present invention.

Furthermore, WO 2016/079170 suggests a method basically corresponding to WO 2013/ 101855 A1, where the polymer of an electron beam (e-beam) reactive moiety is to be linked via a linkage to a reactive group for binding the biomolecules. The reactive group is suggested to comprise maleimide, iodoacetate, bromide, N-hydroxysuccinimide-ester (NHS-ester), anhydride, sulfide, carboxylic acid, aldehyde, or combinations thereof and the linkage can be an ester, an aliphatic chain, a cycloaliphatic chain, an aromatic chain, a heterocyclic compound, a hydrophilic compound, a hetero-aromatic compound, hetero atoms or any combination thereof. A porous membrane coated with a correspondingly modified polymer shall then be incubated with a biomolecule that binds to the porous membrane through reaction with the reactive group to form an activated porous membrane comprising the first biomolecules. Obviously, the biomolecules must be able to react with the reactive group for binding the biomolecules and this may require modification of the biomolecules which can be both bothersome and adversely affect the sensitivity thereof.

Another method of immobilizing antibodies or other reactive substances is to encapsulate them partially. Partial encapsulation can be obtained using porous materials such as hydrogels so that access the body fluids can access the antibodies through the pores of the porous material.

Electron beam assisted grafting for covalent immobilization of enzymes such as trypsin or laccase has been shown in literature (Starke, et al., 2013; Jahangiri et al., 2014; Schulze et al., 2015).

Such "Hydrogels and Their Role in Biosensing Applications" are known e.g. from A. Herrmann et al. in Adv. Healthc. Mater. 2021, 10, 2100062 2100062, where it has been stated that hydrogels play an important role in the field of biomedical research and diagnostic medicine, further stating that hydrogels are emerging as a powerful tool in the context of bioanalytical assays and biosensing and are beneficial for the immobilization and embedding of biomolecules. They are also said to be usable as responsive material, as wearable devices, or as functional material. The authors state that preservation of the native structure of biomolecules is a crucial requirement for feasibility, specificity and sensitivity in biosensing applications and that one of the first systems that used biomolecule entrapment was devised in 1967 by Updike and Hicks, entrapping glucose-oxidase in a gel of polyacrylamide thus increasing the operational stability of the enzyme and simplifying the sensor preparation. The authors also state that there are different approaches for the encapsulation of biomolecules, ranging from size-selective encapsulation to covalent linkage to entrapment of an object which carries the bioprobe. It is stated that the typical advantages of the hydrogel approach over direct immobilization onto substrates include higher loading, better accessibility and a more biological environment provided by the flexible nature of hydrogels and they refer to reports about the encapsulation of biomolecules, particularly enzymes into hydrogels. The authors state that encapsulation of enzymes was found to preserve the native protein conformation and to thereby improve enzyme activity and the authors also refer to super-porous agarose gel that was used in a flow-through electrochemical sensor to immobilize the signal-producing enzymes in larger amounts.

However, while encapsulating biomolecules may help with the activity, a number of problems remain, in particular with respect to the reproducibility of sensitivities. While some variation is acceptable and can be accounted for by calibration, e.g. calibration of batches, any strong variations will cause problems e.g. because sensor non-linearity and the like might become noticeable.

Also, ensuring that the biomolecules become encapsulated may present other obstacles to the use of hydrogels for biomolecule immobilization. For example, diffusion of molecules into the hydrogel will strongly depend on parameters such as cross-linking density and pore size. Accordingly, pore sizes cannot be chosen only in view of the body fluids or the constituents therein - this might adversely affect cross correlations with other substances, in particular as pore size engineering is far from easy. Furthermore, diffusion of the antibodies or other reactive substances will take time during production and might thus add to costs but can also be expected to vary from batch to batch and within a batch, so that reproducibility is low.

Encapsulating the antibodies or other reactive substances in situ while the porous material is produced can also be expected to cause problems. Typically, the porous material is produced from oligomers that are cured. However, both the reactive oligomers and the curing might adversely affect the antibodies or other reactive substances.

For example, curing might be started e.g. by UV irradiation or by adding starter chemicals, both of which might damage the antibodies or other reactive biomolecules. This in turn may affect the overall amount or concentration of antibodies available for detection in the cured material and hence both the sensitivity of a device produced and the reproducibility of detection results, sometimes even within a given batch.

Methods to induce curing other than UV irradiation have been suggested as well. One technique suggested has been electron-beam induced curing for immobilization, comp. e.g. " Electron Beam-Induced Immobilization of Laccase on Porous Supports for Waste Water Treatment Applications" by E. Jahangiri et al. in Molecules 2014, 19, 11860-11882; doi:10.3390/ molecules 190811860. Regarding crosslinking of hydrogels by electron beams, it has been suggested in "A study on the swelling behavior of poly(acrylic acid) hydrogels obtained by electron beam crosslinking" by N. Sheikh et al. in Radiation Physics and Chemistry 79 (2010) 735-739 to use an electron beam for crosslinking PAA homopolymers from an aqueous solution. It is stated that for biomedical use, poly(acrylic acid) would be known to be a suitable model for pH-responsive systems because of the presence of carboxylic acid side groups. Poly(acrylic acid) hydrogel is also reported to have a weak mechanical strength. It is stated that in general, irradiation of a polymer in solution can lead to crosslinking at lower dose than for a solid polymer because of the effect of the radiolysis products of solvent on polymer chains and the lower viscosity of the solution which allows free radical diffusion.

In addition, problems of producing sensors are known to increase if the amount of analytes to be detected in the body fluid is low, if a quantitative measure is needed and/or if the same detection device is to be used repeatedly and/or over a long time, because any adverse effects by previous measurements including fouling due to prior contact with body fluids.

As mentioned before, one analyte of interest is cortisol so a specific problem resides in measuring cortisol in a body fluid, in particular using a biosensor that has a sufficiently long shelf time and can be used repeatedly. These problems become more stringent if a body fluid is selected for being more convenient to provide for a user, even though the analyte concentration in the specific body fluid is lower than in other body fluids and/or more substances causing cross reactions are present during the measurement. One such example is the use of saliva for measuring cortisol.

It would be desirable to provide at least partial improvements to at least some of the problems mentioned above.

It would also be desirable to provide reliable and stable biosensors that allow users to monitor health conditions at home by a non-invasive, pain-free method.

It is an object of the present invention to provide novelties for an industrial application.

This object is achieved by the features of the independent claims. Some of the preferred embodiments can be found in the dependent claims.

Accordingly, what is suggested inter alia by the present invention is a method of producing a biosensor or a part thereof, the biosensor or part thereof comprising a porous polymer, biomolecules adapted to react with an analyte to be sensed with the biosensor, these biomolecules being immobilized by the polymer matrix; a carrier for the polymer matrix, the carrier allowing read-out of reactions of the biomolecules with the analyte, and the method comprising the steps of providing a polymer solution; adding the biomolecules to the solution in defined quantities; dispensing a specific volume of the solution with the added biomolecules onto the carrier; subjecting the solution comprising the added biomolecules and dispensed on the carrier to an ebeam and preferably drying to remove any solvent to immobilize the biomolecules.

This method gives a particularly high reproducibility from batch to batch while at the same time allowing for a simple and cost-effective production of biosensors or biosensor strips. In more detail, by mixing biomolecules with a polymeric solution in a defined, desired quantity, the overall amount of biomolecules in the solutions is high. The biomolecules will typically be in a solution prior to being added to the polymer and, given a known activity (or concentration) thereof, the concentration or overall activity of the biomolecules added to the polymer will depend only on the precision of the volumes mixed. Now, the activity or concentration in the biomolecule solution can be easily determined. Furthermore, precise measurement of a volume is simple. Thus, the overall activity of the biomolecules in the polymer matrix can be easily adjusted precisely; w**hile** the activity of the biomolecules might be altered by e-beam irradiation, this change will be small (typically with an irradiation duration of a few seconds) given that typically, both irradiation intensity and time for irradiation of the mixed solution is rather small. Also, loss of activity can be accounted for easily, as the ebeam irradiation conditions will hardly need to change from batch to batch, even if a different batch of polymer solutions is used. Then, the properties of the matrix obtained from irradiation of the solution are quite reproducible, that is, the porosity of the final product can be reproduced sufficiently precise.

The method disclosed can manufacture either entire biosensors that can be used as a stand-alone device or a part thereof, in particular a sensor strip for use with a read-out device. If a stand-alone device is produced, it might be possible to have a non-electrochemical read-out such as an optical readout by optical determination of e.g. a coloration or an absorption or luminescence. However, the most frequent approach will be by electrochemically detection of reactions, such as by open-circuit potential potentiometry, coulometry, voltammetry,in particular cyclic or square wave voltammetry, and/or amperometry, with a preference for amperometry, in particular chronoamperometry and/or electrochemical impedance. in particular potentiometry, preferably (chrono)amperometry and/or electrical impedance spectroscopy. Reference is had to EP23020052 in this respect, where in particular details about electrode arrangements, measurement, signal determination and evaluation can be found. Note that while detection of glucose in saliva can be considered to be a focus of EP23020052, the measurement methods described therein could be used for any reaction of biomolecules immobilized according to the present invention and are thus neither limited to saliva as body fluid nor to glucose as analyte.

As the polymer in the premix will typically be a solution with a solvent, the solvent will be dried after the irradiation to obtain the biomolecules immobilized in the polymer matrix, the immobilized biomolecules having a quantity precisely controlled by the premix volume and the concentration of the active biomolecules in the premix with the polymer grafting solution used for crosslinked matrix of biomolecule encapsulation/entrapment/immobilization. Despite the simple process, the surface of the matrix will have properties advantageous for typical applications.

It has been found that despite using low energy electron beams, the cross-linking of polymers by e-beam irradiation delivers enough energy for polymer molecules to reconfigure bonds while retaining substantial bioactivity of the immobilized biomolecules due to the low energy of the electron beams. It will be understood that an e-beam basically can be considered "low energy" if on the one hand the activity of the biomolecules is not significantly adversely affected while on the other hand, the e beam energy will still be sufficient to cross-link the polymers.

The overall process is a quick procedure and simple to adapt to new biomolecules as neither additional chemicals nor specific functionalities (on the substrate and/or on the biomolecule) are required. Production speed of biosensors is high as despite exposure of the premix on the carrier to an e-beam radiation having low-energy only, less than a minute of exposure to an e-beam radiation having low-energy will typically be sufficient for a suitable crosslinking process. This allows particularly fast mass production.

Upon irradiation, the premix will form a polymeric hydrogel along with the additional components in the matrix such as the immobilized biomolecules which could be selected e.g. from antibodies, enzymes and other bioreceptors that all can be successfully embedded in the matrix, in particular together with other components such as conductive nanowire and/or particles and/or stabilizer molecules all embedded into the immobilizing matrix and onto the common (typically: polymeric) carrier substrates. It will be understood that such other components can be easily added as well and without significant problems as there is no need to functionalize such additional components either.

As an example, it has been found easy to embed cortisol specific antibodies together with a conductive filler to provide an active biosensing surface. It is obvious that the method is expandable to immobilize anti-cortisol antibodies. In particular, even sensitive antibodies for cortisol or other analytes can be embedded within a hydrogel, which in turn will be grafted onto carriers comprising electrode metal patterns, thus giving to a fully-functioning biosensor for cortisol or other analytes.

A specific advantage of the present invention is that the electron -beam irradiation (or grafting process) of the present invention does not require addition of UV assisting crosslinker such as the N,N methylene bisacrylamide (BIS) and Lithiumphenyl (2,4,6 - trimethylbenzoyl) phosphinate (LAP) needed in the art as UV crosslinker and initiator respectively for polyacrylamide or Poly(2-Hydroxyethylmethylmethacrylate). Rather, neither crosslinker nor initiator are needed in the present invention and hence, no adverse reactions between the biomolecules and any crosslinker or initiator substances will occur.

The invention thus serves as a platform technology for novel biosensors and aside of cortisol antibodies, the use of antibodies specific to other biomolecules is envisioned, e.g. anti-estradiol or anti-testosterone antibodies, allowing development of biosensor for oestradiol or testosterone. It is also possible to use not just antibodies for aptamers so that the invention can also be used for active specific bio-affinity receptors. Also, while below an example will be given with respect to the immobilization in a matrix on electrode metal patterns for electrochemical biosensing, the immobilization strategy will also be beneficial to e.g. optical and/or colorimetric sensing approaches such as ELISA, LFA or other fluorescent, colorimetric or photonic readout-based sensors.

With the invention, it has become easy to maintain the functionality and stability of bioreceptors that are sensitive to handling and would be expected to lose their efficiency with exposure to heat, light or e.g. oxygen, thus allowing for reliable sensing applications compared to conventional techniques using conventional bioreceptor immobilization and giving biosensor with a long shelf life. Furthermore, the overall sensitivity of such biosensors maintaining their activity completely or almost completely during the production process will basically only depend on the accuracy of defining a volume of polymer solution and of precisely pipetting a biomolecule solution into such defined polymer solution volume, thus giving a very high reproducibility as to the quantity and activity of immobilized biomolecules, in particular antibodies. In addition, the biomolecules such as antibodies will be distributed particularly homogeneously within the matrix. Note that rather than pipetting a biomolecule solution into a polymeric solution, a precisely controlled amount of biomolecules could be added to the polymer solution.

In a preferred embodiment, the matrix is formed as a porous hydrogel on the surface of the carrier and the biomolecules are immobilized within the polymer matrix.

Using a porous hydrogel allows easy access of the analyte molecules to the biomolecules immobilized within the matrix. At the same time, porosity can be engineered such that at least molecules in the body fluid significantly larger than the analyte molecules can be prevented to reach the biomolecules or even from penetrating deep into the matrix. Therefore, both a large number of cross reaction and potential fouling can be prevented or reduced.

It is preferred if further to at least one sort of biomolecules added to the solution at least one additional filler selected from conductive fillers, nanoparticles, nanowires, in particular gold nanowires, nanotubes, microspheres, nano/microflakes, 2-dimensional monolayer and/or multilayers made up of metallic or non-metallic materials, and/or a porogen and/or a different sort of biomolecule, in particular one of non-bioactive biomolecule fillers, in particular silk protein, an antibody, an enzyme or a combination of at least one antibody and an enzyme, or an aptamer is added to the solution, the solution being in particular selected from a PEG-matrix building solution or a PVP- matrix building solution.

A plurality of different biomolecules could be used to either provide signals from the biosensor that are less prone to cross-sensitivities, e.g. by providing two different biomolecules that both react with the same analyte giving an improved signal relating to the specific analyte, or to provide two different biomolecules that each react with a different analyte where the two analytes relate to the same physical condition such as a specific form of cancer, an infectious disease, or the like. In this manner, a disease or physical condition can be more precisely detected using "fingerprint" signals relying on a plurality of different sorts of biomolecules. It is possible to have more than two different reactive biomolecules and it is not necessary although preferred that all or at least more than one sort of biomolecules will not be specifically adapted for reaction with or linking to matrix constituents. The biomolecules could be antibodies, aptamers, enzymes or a combination of antibody and enzymes.

Adding bioactive biomolecule fillers such as, but not limited to, silk protein, helps to provide higher thermal stability to the bioactive molecules. These filler proteins can be immobilized along with bioactive molecules.

A PEG- or a PVP- matrix building solution is helpful to prevent fouling and to give a medically safe matrix material that is or can be FDA approved.

Using conductive fillers such as nanoparticles, nanowires, in particular gold nanowires, nanotubes, microspheres, nano/microflakes, 2-dimensional monolayer or multilayers made up of metallic or non-metallic materials helps to generate electrochemical signals if the matrix is to be used on an electrode. It will be understood that this allows for easy improvements of the functionality, in particular for electrochemical sensors as described per se in EP23020052 of the same applicant.

Adding porogens is helpful in engineering the porosity of the matrix to a desired size range. In particular, porogens that can be leached out of the matrix following the irradiation of the mixed solution placed on the carrier are preferred.

The different materials can all be added to the polymer solution and can be incorporated therein, and can in particular be immobilized therein in a single irradiation step (unless intentionally leached out afterwards).

From the above, it will be understood that it is preferred if the biomolecules immobilized within the polymer matrix are larger than the analytes to be sensed and the matrix is produced with a pore size such that the biomolecules added to the solution are essentially retained within the matrix while analytes are allowed to reach the biomolecules. It will be understood that the porosity - that is, the number of pores, their size distribution and the percolation can be engineered inter alia by selection of proper porogens, by selection of e-beam irradiation intensity, irradiating e-beam energy and/or irradiation duration as well as by selecting a suitable polymer solution. It will be understood that e.g. changes in a polymer solution due to variations in the polymer production might have an effect on matrix properties and in particular porosity; parameters such as polymer concentration, crosslinker type and concentration will have an effect on the relative pore size, as the same electron beam irradiation will give a different crosslink density of the hydrogel and a different extension of the relative pore size distribution depending on these parameters. However, it is easy to (re-)adjust the ebeam parameters so that with a given porogen, a porosity yielding a specific behavior of the matrix is obtained. Even though experiments such as determination of swelling and/or retention of either the actual biomolecule or a molecule more easily detectable such as horse-radish peroxidase in a solution such as a redox solution might be needed, any determinations for re-adjusting these parameters are very straightforward, in particular if the respective person understands how an irradiation energy, intensity and duration that is too low or too high adversely affects the properties of the matrix. Thus, any such adjustments can be made easily. Therefore, it will be understood that the porosity and in particular the typical range of pore sizes can be engineered to have specific values.

Accordingly, it is both preferred and possible that the matrix is further produced with a pore size such as to prevent at least a part of proteins in the body fluid from entering the hydrogel, in particular at least a part of those proteins that could cause fouling. Note that the specific pore size needed (if any) might depend on the specific use case, e.g. the specific body fluid to be examined. Where proteins might foul the surface, it is preferable if they are only weakly attached and can be washed off easily, in particular with water or mild detergents or mild solvents such as alcohol. It will be understood that this may depend on the surface of the matrix and thus the matrix material and it should be emphasized again that both PEG and PVP have shown good antifouling character, albeit such antifouling property need not be restricted to PEG and PVP. In this context, it should be noted that the possibility of using the immobilizing polymer matrix itself as an antifouling material reduces the need of additional surface modification to achieve anti-fouling properties and is thus considered advantageous.

Regarding the addition of suitable porogens, in particular porogens of a specific size, gelatin and paraffin microspheres have been successfully used as porogens in the art; reference shall be had in particular to water soluble salts, in particular non-harmful water soluble salts, in particular NaCl crystals as a popular and easy to use porogen.

It is also preferred if the pore size is adjusted to a range of 1 to 100nm. Obviously, since the irradiation and the subsequent crosslinking is a stochastic process, the pore size will of course not be fixed but has a somewhat defined range, with the value given above relating to typical boundaries which however change upon changing the Ebeam parameters for a given solution. For example, when crosslinking more, the average pore size can be expected to become smaller.

Moreover, the immobilization approach of the present invention allows porous surface formation in a manner not possible with chemical and /or adsorption based immobilization approaches. In more detail, the pore size of the hydrogel can be controlled by varying the molecular weight of the polymer; as indicated above, parameters such as polymer concentration, crosslinker type and concentration will have an effect on the relative pore size. This allows in particular to pre-select a polymer with an appropriate molecular weight and to then fine-tune pore sizes by setting e-beam irradiation parameters adequately. In addition to that, a template method or particle leaching as a well-established approach to control the pore size of hydrogels by use of a porogen is feasible as well, even though in a number of cases, addition of porogens might not be necessary. When porogens are used, a porogen of a well-defined size is dispersed with the hydrogel premix including the biomolecules (and fillers, if any) and the hydrogel premix is allowed to solidify. After solidification of such hydrogel premix, the porogen is dissolved away or leached out using a selective solvent, leaving behind a porous network. For example, owing to its controlled size and cost, NaCl has been a popular choice of porogen material which can also be used current method.

However, despite the possibilities offered by the present invention, reducing the pore size might still be somewhat restricted. This however actually can be considered an advantage as for example the pore size of a matrix may remain significantly larger than the size of target molecules such as cortisol while at the same time restricting entry of bigger molecules. More precisely, the average pore size can be smaller than the typical size of the antibodies, leading to an effective encapsulation/immobilization of antibodies which are preferred biomolecules for sensing analytes. Accordingly, electrodes functionalised for biosensing such as for sensing cortisol can be used with biofluids such as saliva, sweat or blood where bigger molecules can be expected to be present. Thus, in particular cortisol can easily diffuse into the matrix while large unwanted molecules can be prevented. e.g., larger proteins from saliva, and while leaching tests indicate that biomolecules immobilized within the hydrogel can withstand washing/solvent flow and remain in the location where they are immobilized or at least close thereto. This in turn can be exploited to wash and remove any non-specific binding and unbound molecules both after production and in case of repeated use.

As can be seen from the above, it may be preferred though not absolutely necessary if a porogen is added to the solution and is leached out following irradiation of the solution dispensed on the carrier, and/or if the molecular weight of polymers and/or a crosslink density is selected such as to give the required porosity for retaining the immobilized biomolecules while retaining sufficient access thereto. As will be understood from the above, the porogen will have a well-defined size defined in view of a pore size distribution wanted, will be in a precisely defined amount in the hydrogel premix and will be allowed to solidify. After the irradiation and once any solvent is dried to immobilize the biomolecules in the polmer matrix in a precise quantity, the porogen can be leached out.

It should be understood that typically, it will be preferred if the solution on the carrier is exposed to an e-beam radiation sufficient for a crosslinking process for a duration of less than two minutes, preferably less than one minute.

It is also preferred if the parameters of the e-beam and in particular the energy (=electron wavelength) are such that the activity of the biomolecules when exposed to an e-beam radiation outside a matrix building solution is reduced by less than 30%, preferably less than 20%, in particular less than 10%.

It is noted that corresponding tests can be carried out rather easily. In more detail, the energy that will reduce the biological activity of the biomolecules when exposed to an e-beam radiation outside a matrix building solution can be determined by determining the activity of a solution of the biomolecules to be added in specific quantities to the polymer solution during execution of the invention; exposing the solution of biomolecules as added in specific quantities to the polymer solution during execution of the invention to an e-beam having the duration, intensity (electron current strength) and energy (= electron wavelength or electron acceleration voltage) of the e-beam considered to be used later on for cross-linking the premix solution (that is, for matrix building). Then, the activity of solution after irradiation is determined and compared to the pre-radiation activity. Note that a loss of activity observed in this manner will generally be smaller than a loss of activity once the biomolecule is protected by the matrix building up during irradiation. However, a loss of activity of unprotected biomolecules larger than 30% may result in significant damage to biomolecules even prior to building up the matrix and this may be disadvantageous because the molecules damaged might react in an unknown way, probably causing interferences with desired reactions of the undamaged biomolecules. Therefore, even lower boundaries for maximum reduction of activity are preferred. Note that at the same time, high intensities, high energies and/or long durations might adversely affect the resulting matrix, e.g. because the matrix is not stable enough after "harsh" crosslinking resulting from such parameters. In general, it should be understood that for the typical sensor geometries, polymers and biomolecules, a duration of exposure of less than a minute is sufficient for the crosslinking process and that the e-beam radiation should be low-energy.

Regarding the term "low-energy" as used herein, it will be understood that on the one hand, the matrix is produced by ebeam irradiation from the premix solution. For this to happen, the radiation must be strong enough to penetrate deep enough into the drop of premix solution dispensed onto the carrier-as the penetration. In more detail, the premix solution will absorb some of the radiation while it passes and if this absorption is too strong, the irradiation dose will vary strongly across the drop, e.g. when entering into the drop opposite of the carrier, from the entrance point to the carrier. Such variation will be smaller if the e-beam energy (electron wavelength or acceleration voltage) is somewhat higher. It will be understood that the overall absorption along the optical path length corresponding to the size of a dispensed drop of premix solution should not be higher than 15%, preferably not higher than 10%, in particular less than 5% and in the most preferred cases will be less than 2%. These values give a sufficiently homogenous matrix, with the smaller values obviously being preferred if the matrix is to become more homogenous. For lower absoption, the energy should be higher. As the skilled person will thus be able to measure or calculate absorptions for different energies and different premix solutions (having different densities), these values give a good understanding of a reasonable lower energy limit for a "low energy" beam. On the other hand, it will also be understood that higher acceleration voltages for the electron beam (read: higher energy-beams) might at the same time cause more damage to the biomolecules. Here, it is to be understood that such damage must be limited to warrant the use of the term "low-energy". As also stated elsewhere in the application, it is preferred if the ebeam energy (=electron wavelength) is such that the activity of the biomolecules when exposed to an e-beam radiation outside a matrix building solution is reduced by less than 30%, preferably less than 20%, in particular less than 10%. Accordingly, the term low-energy can be construed to have at least an energy where absorption across the drop dispensed onto a carrier is not higher than 15%, preferably not higher than 10%, in particular less than 5% and in the most preferred cases will be less than 2% while at the same time, the electron beam energy is not higher than that which causes the activity of the biomolecules when exposed to an e-beam radiation outside a matrix building solution to be reduced by no more than 30%, preferably not more than 20%, in particular by less than 10%.

To obtain an even better understanding of the term "low-energy" in this context, it should be noted that even for identical solutions, optimum parameters might be machine-dependent, e.g. because of errors in an energy calibration. However, as e-beam irradiation is done to cross-link the polymers to build up the matrix, it is straightforward to determine whether certain parameters lead to good cross-linking of a solution or not. In more detail, the respective polymer solution to which an active biomolecule or a substitute for such biomolecule has been added can be placed onto a carrier and subjected to irradiation with different parameters. Then, the resulting matrix can be placed in a solution to observe swelling or signs of damage such as washing out of the biomolecules; then, the activity of the biomolecules in the matrix can be determined. Note that it is not necessary to do this with the actual biomolecules where only a general understanding of the cross-linking is needed. This allows e.g. to use UV absorbing or fluorescent substitutes instead of the actual biomolecules, thus greatly speeding up the determinations. When doing this for a number of different parameters covering sufficiently wide ranges, uncomplete cross-linking resulting in material that can be washed off easily, sufficient cross-linking giving good activities suitable for use and harsh crosslinking that results in damage during the swelling will be observed.

As an example, in a practical implementation, for different Ebeam Dosages and different electron accelerating voltages, the following observations have been made:
A dosage of 50kGy with an ebeam of 80kV acceleration was found to give very low crosslinking and a hydrogel which was not mechanically stable. This setting can thus be termed a low crosslinking setting.

A dosage of 50kGy with an ebeam of 150kV acceleration was found to give a hydrogel which is mechanically stable, yielding optimum results. This setting can thus be termed a standard crosslinking setting.

A dosage of 100kGy with an ebeam of 180kV acceleration was also found to give a hydrogel which is mechanically stable, yielding again optimum results, so this setting can also be termed a standard crosslinking setting.

A dosage of 100kGy with an ebeam of 180kV acceleration was found to give some visual damage to the substrate and the hydrogel, which was mechanically stable, although having a performance which was not optimum due to the biomolecules being subjected to an irradiation too strong. This setting can thus be termed a medium harsh setting.

A dosage of 150kGy with an ebeam of 200kV acceleration was found to give considerable visual damage to both the substrate and the hydrogel, which again was mechanically stable, although again having a performance which was not optimum due to the biomolecules being subjected to an irradiation too strong. So, this setting can be termed a "harsh" setting.

As can be seen from the above, a range of very good settings exist, which makes selection of suitable parameters more simple. It should be mentioned that from experimental evidence, a window of energy from 80kV - 150kV was found to be 'safe' provided the dosage was kept <100kGy. When increasing the energy, it is currently considered better to reduce the dosage in order to obtain suitable settings. For example, it has been noted that while 200kV and 50kGy seem to give acceptable results okay, a setting of 200kV and 100kGy has been observed to degrade the polymer.

It is also preferred if the carrier surface is pretreated for improved wettability by the solution, in particular by at least one of corona discharge treatment, atmospheric plasma treatment, vacuum oxygen plasma treatment or UV-Ozone treatment, in particular by spatial selective treatment defining areas on the carrier that will contain the solution onto a desired location and prevent any flow or movement thereof on the carrier until e-beam irradiation.

A person skilled in the art will understand that this helps binding the matrix to the carrier. This holds even if the carrier surface is provided with electrodes. In a preferred embodiment, such electrodes can have a metal pattern such as chequered or sieve-like with non-metallized areas therebetween, so that the matrix covering both metallized and non-metallized areas. This will help to bind the matrix to the carrier even if the binding is only improved in the non-metallized areas. It should also be understood that such treatment not only increases the wettability of the carrier surface, rendering it hydrophilic for better spreading of the immobilization/grafting (premix) solution, but that the treatment also helps with cleaning and disinfecting the carrier surface.

So, in one preferred step, surface activation can be carried out using atmospheric plasma. It will be understood that one particularly preferred material for the carrier is PMMA, as it can be plasma treated without problems and can also be provided easily with metal patterned electrodes.

It should also be understood that it would be possible to functionalize additional electrodes directly, that is in a matrix free manner with suitably functionalized antibodies. Different electrodes could be provided on the same carrier for different premixes, each premix containing one or more sort of biomolecules, with the different premixes differing in the sort and/or amount of these biomolecules and/or in the resulting porosity as obtained by e.g. different porogens. Premixes might also differ in polymer weights of the respective polymer solution- the skilled person will understand that also, different amounts and/or types of fillers, of conductive materials and so forth might be used for such different premixes. Using a multitude of different premixes on one sensor allows e.g. to build multi-analyte biosensors where signal evaluation is more simple than in a case where several biomolecules for different analytes are mixed into the same premix.

Protection is also sought for a cortisol sensing biosensor or parts thereof produced as indicated above and /or in one of the method claims, wherein the polymer matrix is a hydrogel adapted for use with at least one of blood, sweat, tears, urine or saliva, in particular for saliva and the biomolecules immobilized in the polymer matrix are anti-cortisol antibodies, in particular monoclonal anti-cortisol antibodies.

Thus, while the invention suggests to immobilize biomolecules in general, in particular antibodies, an embodiment considered particularly important at the date of filing is cortisol-related biomolecules, in particular monoclonal anti-cortisol antibodies immobilized within a hydrogel that is in turn provided on electrodes for biosensing application. Nonetheless, many variations are possible while using the overall same inventive immobilization approach.

While the present invention can be used for a large variety of analytes, measuring Cortisol is particularly attractive. First of all, Cortisol is a hormone that spikes in response to the stress in an individual. By measuring its levels, stress - both physical and emotional - experienced by a person can be determined. As chronic stress is significantly reducing life quality and also induces illness, it is helpful to monitor stress frequently, in particular frequently enough before reactions to stress become harmful to the individual. Accordingly, measuring Cortisol is helpful in mitigating stress. Although this has been know for quite some time, use of prior art cortisol detection systems has been time consuming and did require professional assistance instead of being user-friendly.

In contrast, the present invention allows for a fast and reliable way to measure Cortisol using saliva as body fluid and allowing to measure current Cortisol level even for unexperi-enced users.

In this context, it should be noted that the pore size of the typical matrix can be engineered significantly larger than the size of the target cortisol molecules, so that easy diffusion of cortisol into the matrix is allowed while at the same time restricting entry of bigger molecules typically found in saliva. Thus, a functionalized electrode for cortisol biosensing can be used for biofluids such as saliva, sweat or even blood.

In one preferred embodiment, additional filler materials such as gold nanowires will be used to provide a conductive matrix; as the overall amount of gold needed is small, this will not increase costs significantly and adverse reactions with the immobilized biomolecules are unlikely. Furthermore, a thermal stabilizer such as a protein, in particular a silk protein, can be added. This helps to maintain sensitivity even over an extended shelf life time. Other fillers can also be used without necessitating a modification of the biomolecules. As the presence of some of the fillers may alter the pore size, such change should be accounted for.

It is noted that leaching tests have indicated that biomolecules and the hydrogel can withstand washing and/or solvent flow so that the biomolecules remain in the location where they are immobilized. This can be exploited to wash and remove any non-specific binding and unbound molecule. Also, the matrix surface can be selected from materials that prevent fouling and are medically safe; in particular, PEG and PVP are usable as polymers for the polymer matrix in a cortisol biosensor. Therefore, even repeated use of a sensor strip or the like becomes feasible.

If the biomolecules in the cortisol biosensor are anti-cortisol antibodies, the overall (porous) matrix of the biosensor might comprise anti-cortisol antibodies, conductive filler material and stabilizing molecules, as well as the hydrogels itself made of common biocompatible polymers, in particular PEG or PVP. The energy and irradiation intensity of the electron beam for irradiating the corresponding premix can be selected such that for producing a Cortisol biosensor low energy electron beam grafting (e-grafting) is employed, varying the process parameters so as to gain control over the crosslinking and consequently the pore size of the hydrogels.

It is noted that the biosensor obtained can be used in particular as a sensor strip for a read-out device as described in EP23020052.9 (unpublished on the priority day of the present application). This document is fully incorporated herein for reference in particular with respect to any aspect relating to preferred measurement methods, in particular with respect to electrochemical measurements, suggesting inter alia a read-out device comprising an interface adapted to obtain signals indicative of an analyte level of an analyte in a body fluid from a carrier adapted to receive in use the body fluid and to react in a detectable manner with the analyte and/or one or more concomitant substances in the body fluid; and a signal processing stage adapted to process the signals in a manner allowing determination of the level of the analyte in the body fluid; wherein the interface is adapted to obtain at least part of the signal as a time series of signal strengths from the carrier after receipt in use of one type of body fluid selectable from at least two different types with each type containing the analyte in a determinable amount, and the signal processing stage is adapted to assess the analyte level from the time series of signal strengths for either of the types of body fluid received on the carrier.

EP23020052 also suggests that the interface is adapted obtain the signals from the carrier optically and/or electrochemically, preferably by chronoamperometry and/or by electrical impedance spectroscopy. It will be understood that this is useful for a biosensor produced according to the present invention as well.

Furthermore, EP23020052 also suggests that the group of at least two different types of body fluid comprises at least one of (blood, urine, saliva) and preferably at least two of (blood, urine, saliva), in particular blood and saliva and it will be understood that this is applicable with a biosensor produced according to the present invention as well.

Furthermore, EP23020052 suggests that the signal processing stage can be adapted to store coefficients and/or parameters for at least two different models for assessing the level of analyte in response to the parameters, with each model relating to a different type of body fluid, the signal processing stage being adapted to select the appropriate model in view of the time series of values and/or in view of additional signals being obtained and it will be understood that this is applicable with a biosensor produced according to the present invention as well.

Furthermore, EP23020052 suggests that the signal obtained is digitized and the signal processing stage is adapted to process a time series of digital values and it will be understood that this is applicable with a biosensor produced according to the present invention as well.

Furthermore, EP23020052 suggests that the signal processing stage is adapted to evaluate the time series locally such that an indication of the level of analyte can be output and it will be understood that this is applicable with a biosensor produced according to the present invention as well.

Furthermore, EP23020052 suggests that the signal processing stage can be adapted to process a time series comprising at least 5 samples, preferably in particular at least 10 samples, particularly preferably at least 20 samples, taken over a period of time sufficient for observing the onset of a signal increase, in particular over a period of at least 2, preferably at least 5, in particular at least 10 seconds, and comprising samples obtaining following a maximum of a signal strength value and/or having a resolution after digitalization of at least 8, preferably at least 10 in particular at least 12 bit and it will be understood that this is applicable with a biosensor produced according to the present invention as well.

Furthermore, EP23020052 suggests that the signal processing stage can be adapted to assess the level of analyte in a quantitative manner and it will be understood that this is applicable with a biosensor produced according to the present invention as well.

Furthermore, EP23020052 suggests that the interface can be further adapted to read out calibration information from the carrier and the signal processing stage is adapted to determine a plurality of parameters from the time series of digital signal values and to assess the level of analyte in response to both the parameters determined and the calibration information and it will be understood that this is applicable with a biosensor produced according to the present invention as well.

Furthermore, EP23020052 suggests that the analyte can be glucose or a concomitant indicator of glucose concentration and it will be understood that this is applicable with a biosensor produced according to the present invention as well.

Furthermore, EP23020052 suggests that the signal processing stage can be adapted to store parameters and/or coefficients for at least two different models for assessing the level of analyte in response to the parameters, with each model relating to a different type of body fluid, the signal processing stage being adapted to select the appropriate model in view of the time series of values and/or in view of additional values being obtained and it will be understood that this is applicable with a biosensor produced according to the present invention as well.

Furthermore, EP23020052 suggests that a preferred read-out device can be adapted to obtain signals from disposable carriers and it will be understood that this is applicable with a biosensor produced according to the present invention as well.

Furthermore, EP23020052 suggests that a preferred read-out device can have an interface to a smart device, in particular a smart phone, smart watch or a laptop and it will be understood that this is applicable with a biosensor produced according to the present invention as well.

Furthermore, it will be understood that the method suggested and disclosed herein can be used to produce a disposable carrier as a part of a biosensor, the disposable carrier being adapted for use with a read-out device as described in EP23020052, wherein the carrier can be adapted to react with an analyte or a concomitant substance in the at least two types of body fluid and the interface is adapted to obtain the signal optically and/or electrochemically, preferably by chronoamperometry or by electrical impedance spectroscopy and it will be understood that this is applicable with a biosensor produced according to the present invention as well.

The immobilization method described herein could also be used to produce a disposable carrier as described in EP23020052 in that it is having electrodes for chronoamperometry, the electrodes being coated with the matrix comprising biomolecules as reagents for glucose detection, the disposable carrier also comprising additional electrodes for the determination of a body fluid type by electrical impedance spectroscopy and the carrier having associated therewith or carrying calibration information relating to at least types of body fluid with one body fluid being blood and it will be understood that this is applicable with a biosensor produced according to the present invention as well.

Details for such read-out devices, parts thereof and their use can be found in EP23020052 fully incorporated herein by way of reference and it will be understood that the methods of signal evaluation described therein are helpful to obtain meaningful measurement with devices produced according to the present invention described herein, even if the biosensor or part thereof described in here should be intended for use with only one specified body solution such as for example only saliva or only urine. This being said, the description and features of such methods of signal evaluation described in EP23020052 are fully incorporated herein by way of reference.

The invention will now be described by way of non-limiting examples hereinafter, referring to the figures showing in
Fig 1 -5 steps and/or intermediate products in producing a biosensor or part thereof according to the present invention, namely
   Fig. 1 the carrier substrate without electrode;
   Fig. 2 the carrier substrate after electrode fabrication;
   Fig. 3 the step of precision-dispensing the premix onto the carrier;
   Fig. 4 the step of a short ebeam irradiation of a premix precision-dispensed onto the carrier;
   Fig. 5 the carrier with the immobilzed sensing surface;
Fig 6 -11 Penetration depths for different dosages and beam energies ;
   note
   that the X and Y-axis differ for the different figures and
   that, while the absorption at a given depth is constant for a given beam energy, the overall dose reaching a specific depth will of course depend on the overall irradiation dose.

Hereinafter, the production of a biosensor strip useable as a part of a cortisol biosensor will be described. The biosensor will be usable for detection of cortisol in saliva and the biosensor strip produced will comprise a porous polymer; biomolecules which in the specific embodiment shown are suitable for reaction with cortisol as an analyte to be sensed by said biosensor, said biomolecules being immobilized by the polymer matrix; a carrier for the polymer matrix, the carrier allowing read-out of reactions of the biomolecules with an analyte. The biosensor strip can be used e.g. with a read-out device as described in EP23020052.9.

For producing such a biosensor usable as a biosensor detector strip for a chronoamperometric determination of cortisol as an analyte, first, PMMA foil to be used as a carrier is provided (Fig. 1) with a suitable metal electrode pattern (Fig.2) , in particular a chequered metallization pattern (not shown). Then, the metallized PMMA foil is surface activated using atmospheric plasma (not shown), although e.g. UV-Ozone treatment or vacuum oxygen plasma could also be used to clean the surface and to render it hydrophilic for better spreading of immobilization/grafting solution in a subsequent step. Note that such plasma pretreatment is preferred but not deemed absolutely necessary.

A solution of the biomolecules suitable for reaction with the analyte is provided and aliquots thereof are subjected for one minute to ebeam irradiation having different electron energies. For each ebeam energy, the decrease of reactivity of the solution by irradiation is determined and the maximum allowable energy is set such that the decrease is lower than 10%, this being considered a suitable low-energy beam irradiation in the context of the present invention.

Thereafter, a PEG polymer is provided characterized by the manufacturer by its Dalton molecular weight of PEG polymer.

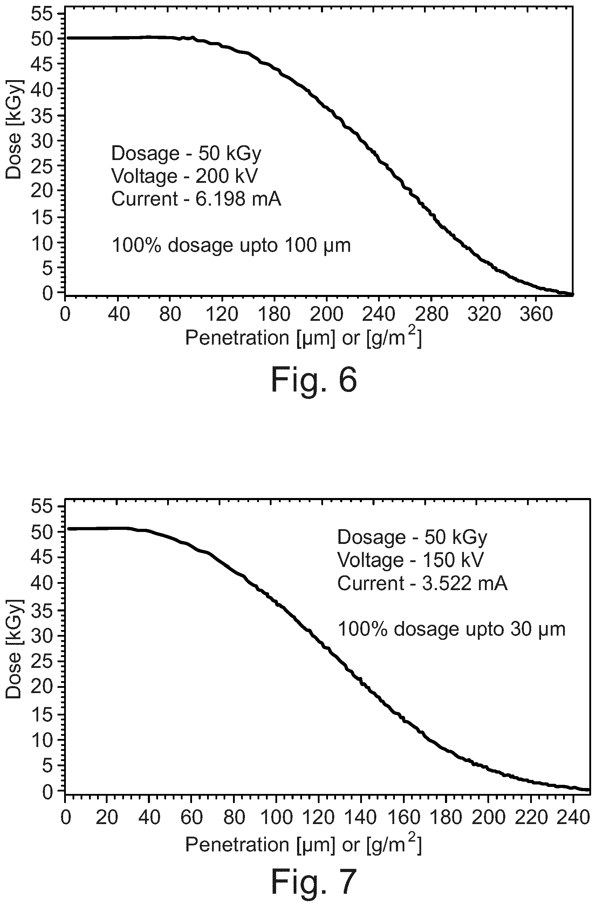

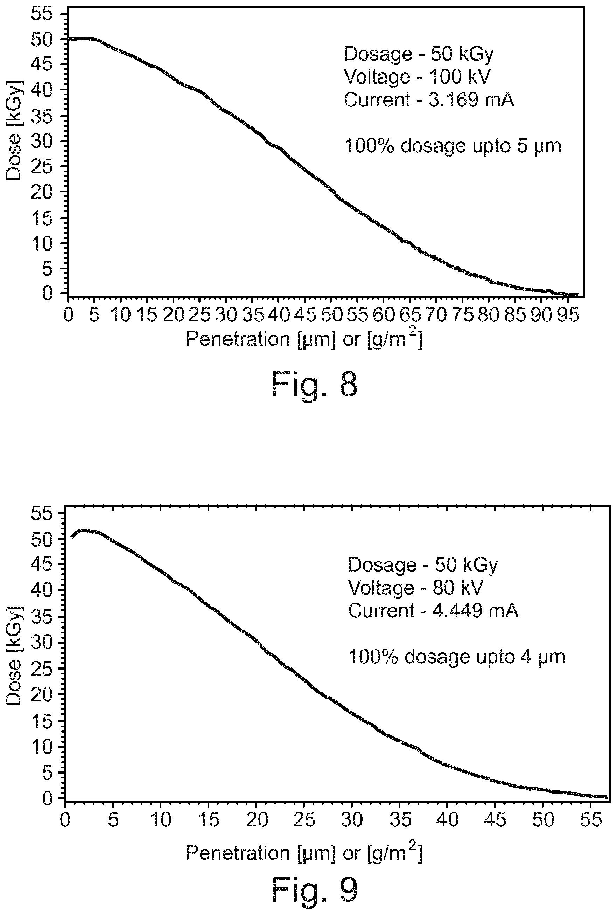

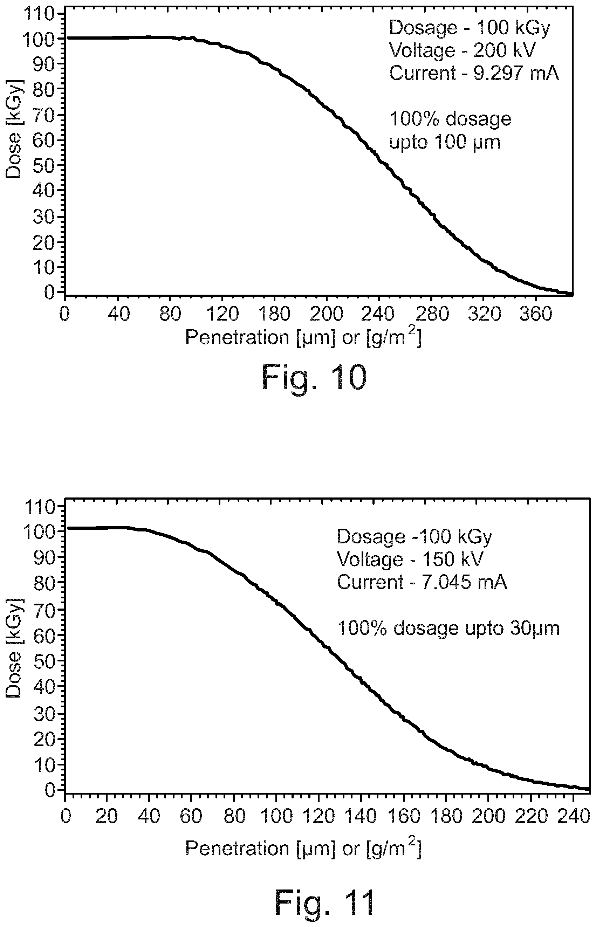

For the given molecular weight of the PEG polymer, ebeam-irradiation intensity and duration parameters are then determined. First, the overall absorption in the respective polymer solution is calculated as a function of penetration depth into the solution. Note that the respective polymer solution will have a specific density so that a specific absorption can be calculated. The calculations are shown in Fig. 6-11.

Then, using the results as a basis for initial intensity and energy parameters, polymer solution is mixed with an indicator that allows to determine cross-linking quality after irradiation. As this indicator need not be the same biomolecules used in the actual biosensor, horseradish peroxidase or any other easily available and common biomolecule can be used.

Defined volumes of the solution mixed with the indicator are then placed onto the electrode patterns and irradiated with ebeams with the different intensities and for different durations, but with energies low enough to not substantially harm the actual biomolecules as determined in the previous tests.

After these irradiations, the matrices obtained are dried and then subjected to a swelling test by placing the strips in a redox solution for a fixed time such as 15 min. Thereafter, a visible control is carried out whether or not the strips look damaged and for the non-damaged looking strips (with damage indicating ebeam parameters giving an irradiation which is too harsh) and a determination is made whether or not the indicator has been washed out or washed out partially to an extent too large (which indicates insufficient cross-linking and thus durations that are too short or intensities that are insufficient). This can be done e.g. by colorimetric assessment of the HRP concentration remaining in the swelling matrix or found in the swelling solution. It will be found that there is an optimum irradiation intensity and such optimum can be found easily as described. Accordingly, e-beam parameters yielding a suitable matrix can be determined for the PEG polymer with the given Dalton molecular weight.

Note that it is possible to do this determination for polymers differing e.g. with respect to molecular weight and to determine a Dalton molecular weight preferred in that the retention of indicator is particularly large for a given acceptable ebeam irradiation intensity and duration.

Thereafter, the actual premix is prepared, using precisely controlled volumes of the PEG polymer solution, of the biomolecules and adding, as deemed necessary, porogens, conductive fillers such as gold nanowires and/or thermal stabilizers such as large inert silk protein.

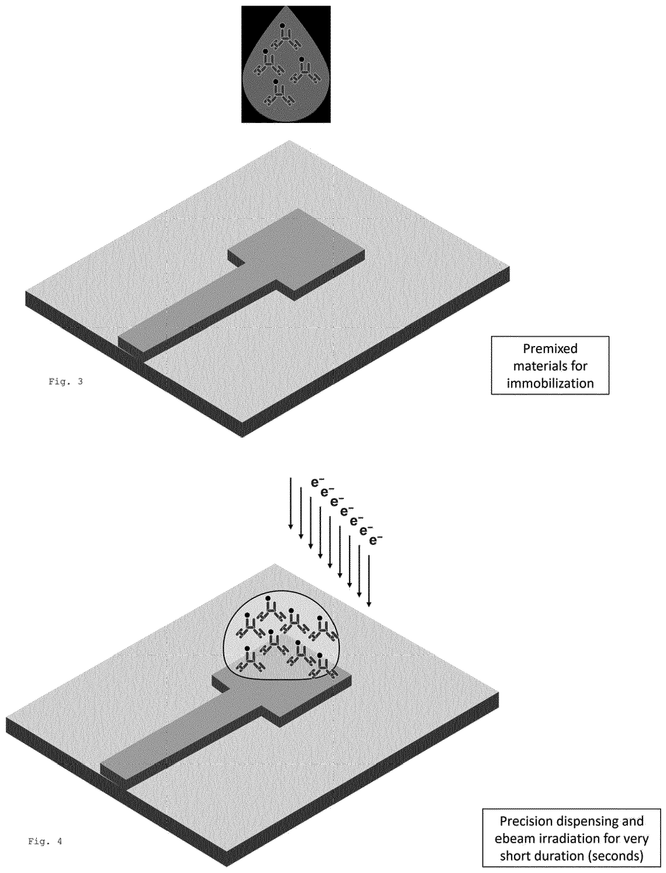

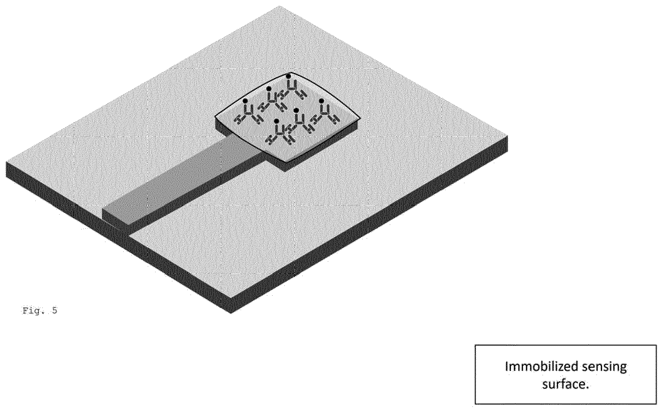

The premix is placed on the prepared, patterned and plasma-treated carrier (Fig. 3) and irradiated with an ebeam having the parameters determined (Fig. 4). In this manner, immobilization/entrapment of the antibodies is achieved in the crosslinked matrix having a pore size that can be made smaller than the typical size of the antibodies, thus being to an effective encapsulation/immobilization (Fig. 5).

After irradiation, any solvent is dried and, if porogens have been used, these are leached out from the strips.

Then, the biosensor is ready for use. It can be shown that the sensitivity of sensors within a given batch and from batch to batch does hardly show any variation and that the shelf-life, that is the time until deterioration, is long.

As indicated above, electron beam assisted grafting is used for the immobilization for specific biomolecules, in particular for antibodies, in a matrix. In contrast to reports in the literature suggesting to covalently immobilize enzymes such as trypsin or laccase by electron beam assisted grafting, the present invention and its specific methods allows to immobilize even antibodies and do so without specific adaption of the antibodies, even though an e-beam irradiation can be expected to be potentially detrimental to the biological structures and thus their function.

Accordingly, antibodies, in particular monoclonal antibodies, enzymes and other biomolecules acting as bioreceptors can be successfully embedded in a polymeric hydrogel with the novel method, along with any additional components in the matrix required to produce a stable electrode such as conductive nanowire or particles, stabilizer molecules and common polymeric substrates can be used as carriers, in particular by using atmospheric plasma and low energy electron beam grafting (e-grafting) technique whilst retaining the bioactivity of the bioreceptors using the same overall inventive immobilization approach for a large variety of biosensing applications, in particular when using a suitable detection and handling of electrochemical signals..

Therefore, the new method of the invention is particularly attractive as it is bascally a one-step procedure where neither chemicals need to be added nor specific functionalizations (on the substrate or the biological molecules) are required. Thus, a large number of antibodies can be expected to be immobiliza-ble without modification by the suggested embedding within a (hydrogel-) matrix. Given that such hydrogel can easily be made conductive, porous and selective at the same time and can also be grafted easily onto electrodes, fast development of new biosensors such as a fully-functioning cortisol biosensor is allowed.

More generally, the invention provides many performance and economic benefits over the convention immobilization methods, particularly for biosensing applications but not limited to that. In particular, the invention will serve as a platform technology for biosensors. Thus, instead of the cortisol antibodies specifically mentioned above, other antibodies specific to other biomolecules can be used such as anti-estradiol or anti-testosterone antibodies for specific biosensors development for oestradiol and testosterone respectively. Also, antibodies for aptamers could be used as well as active specific bio-affinity receptors. Furthermore, whereas an embodiment was described which gave a specific immobilization on electrodes for electrochemical biosensing, the immobilization strategy would also provide the same benefits to optical and colorimetric sensing approaches such as ELISA, LFA or other fluorescent, colorimetric or photonic readout-based sensors.

## Claims

1. A method of producing a biosensor or a part thereof,
the biosensor or part thereof comprising
a porous polymer;
biomolecules suitable for reaction with an analyte to be sensed by said biosensor, said biomolecules being immobilized by the polymer matrix;
a carrier for the polymer matrix,
the carrier allowing read-out of reactions of the biomolecules with an analyte;
the method comprising the steps of
providing a polymer solution;
adding the biomolecules to the solution in defined quantities;
dispensing a specific volume of the solution with the added biomolecules onto the carrier
subjecting the solution comprising the added biomolecules and dispensed on the carrier to an ebeam
and preferably to drying to remove any solvent to immobilize the biomolecules.

2. The method according to claim 1, wherein
the matrix is formed as a porous hydrogel on the surface of the carrier
and
the biomolecules are immobilized within the polymer matrix.

3. The method according to the previous claim, wherein
further to at least one sort of biomolecules added to the solution
at least one filler selected from
conductive fillers, nanoparticles, nanowires, in particular gold nanowires, nanotubes, microspheres, nano/microflakes, 2-dimensional monolayer or multilayers made up of metallic or non-metallic materials,
and/or a porogen
and/or an additional, different sort of biomolecule,
in particular one of
non-bioactive biomolecule fillers, in particular silk protein, an antibody, an enzyme or a combination of antibody and enzymes, or an aptamer is added to the solution,
the solution being in particular selected from a PEG- and a PVP- matrix building solution.

4. The method according to the previous claim, wherein
the biomolecules immobilized within the polymer matrix are larger than the analytes to be sensed
and the matrix is produced with a pore size such that
the biomolecules added to the solution are essentially retained within the matrix
while
analytes are allowed to reach the biomolecules,

5. The method according to the previous claim, wherein the the matrix is further produced with a pore size such as to prevent at least a part of proteins in the body fluid from entering the hydrogel, in particular at least a part of those proteins that could cause fouling.

6. The method according to the previous claim, wherein the pore size is adjusted to a range of 1 to 100nm.

7. The method according to the previous claim, wherein
a porogen
is added to the solution
and
is leached out following irradiation of the solution dispensed on the carrier,
and/or the molecular weight of polymers and/or a crosslink density is selected such as to give the required porosity for retaining the immobilized biomolecules but retaining sufficient access thereto.

8. The method according to any of the previous claims, wherein the solution on the carrier is exposed to an e-beam radiation sufficient for a crosslinking process for a duration of less than two minutes, preferably less than one minute.

9. The method according to any of the previous claims, wherein the parameters of the e-beam are such that the activity of the biomolecules when exposed to an e-beam radiation outside a matrix building solution is reduced by less than 30%, preferably less than 20%, in particular less than 10%.

10. A method according to one of the previous claims, wherein the carrier surface is pretreated for improved wettability by the solution,
in particular by at least one of
corona discharge treatment,
atmospheric plasma treatment
vacuum oxygen plasma treatment
or
UV-Ozone treatment,
in particular by spatial selective treatment defining areas on the carrier that will contain the solution onto a desired location and prevent any flow or movement thereof on the carrier until e-beam irradiation.

11. A method according to one of the previous claims, wherein the carrier surface is provided with electrodes, in particular a metal patterned electrodes, particularly preferred chequered or sieve-like patterned electrodes having non-metallized areas therebetween, the matrix covering both metallized and non-metallized areas.

12. A cortisol sensing biosensor or part thereof produced according to claim 1,
wherein the polymer matrix is a hydrogel adapted for use with one of blood, sweat, tears, urine or saliva, in particular for saliva
and
the biomolecules immobilized in the polymer matrix are anti-cortisol antibodies, in particular monoclonal anti-cortisol antibodies.
